# EUROPEAN PATENT APPLICATION

(11) **EP 3 875 577 A1**
(43) Date of publication of application: **08.09.2021**
(21) Application number: 19880216.7
(22) Date of filing: 28.10.2019
(51) Int. Cl.: C12N 1/20, C12N 1/38, C12N 1/04, A23L 29/00, A23L 33/135

(54) **USE OF CYSTEINE OR SALT THEREOF FOR CRYOPROTECTING LACTIC ACID BACTERIA**

(30) Priority: 30.10.2018 KR 20180131242; 23.04.2019 KR 20190047299; 25.10.2019 KR 20190134072
(71) Applicant: CJ Cheiljedang Corporation, Seoul 04560 (KR)
(72) Inventor: SHIN, Dong Joo, Seoul 06802 (KR); KIM, Yeo Jin, Seoul 04718 (KR); KIM, Hyung Cheol, Yongin-si, Gyeonggi-do 16844 (KR); PARK, Hong Wook, Seoul 06219 (KR); BAE, Su Jin, Suwon-si, Gyeonggi-do 16508 (KR)
(74) Representative: Cabinet Laurent & Charras
(86) International application number: PCT/KR2019/014257
(87) International publication number: WO 2020/091334

(57) **Abstract**

The present application relates to use of cysteine or a salt thereof for the protection, growth acceleration and/or stability improvement of lactic acid bacteria.

## Description

### TECHNICAL FIELD

The present application relates to an active ingredient for cryoprotecting lactic acid bacteria, and utilization/application of the active ingredient.

### BACKGROUND ART

Lactic acid bacteria are also called lactobacillus, and are important bacteria which are also used as a medicine for intestinal disorders by preventing abnormal fermentation caused by various germs while living in the intestines of mammals. However, in order to exert the effect of the bacteria, it is necessary to take a much higher amount of lactic acid bacteria than the amount previously taken as food such as yogurt. Therefore, a method for separating only lactic acid bacteria and easily eating the lactic acid bacteria in the form of a powder or a capsule has been popularized. However, when the lactic acid bacteria are made into the form of a powder or a capsule, many lactic acid bacteria die during a long-term distribution process, and thus there is a limitation in that the lactic acid bacteria cannot exhibit an original physiologically active function.

A method such as gelatin coating (Champagne et al., Food Research International, 29, 555 to 562 (1996)) has been performed in order to maintain the physiological activity, but the development of a method by which the survival rate and preservation stability of lactic acid bacteria can be further increased is currently required.

### DISCLOSURE OF THE INVENTION

### TECHNICAL PROBLEM

An object of the present application is to provide: a composition for cryoprotecting lactic acid bacteria or a culture medium for cryoprotecting lactic acid bacteria, which protects lactic acid bacteria from being frozen and imparts high stability such as thermal stability to the lactic acid bacteria even after being freeze-dried to be powdered; and a method for producing a lactic acid bacteria preparation using the same.

Moreover, another object of the present application is to provide: a method which is for accelerating the growth of lactic acid bacteria, and by which the growth of the lactic acid bacteria is accelerated to show excellent productivity of the lactic acid bacteria; and a lactic acid bacteria culture medium for excellently accelerating the growth of the lactic acid bacteria.

### TECHNICAL SOLUTION

In order to achieve the objects, one aspect of the present application provides a composition which is for cryoprotecting lactic acid bacteria and contains cysteine or a salt thereof as an active ingredient.

Another aspect of the present application provides a method for producing a lactic acid bacteria preparation, the method including: mixing lactic acid bacteria with the composition for cryoprotecting lactic acid bacteria; and powdering the mixture.

Still another aspect of the present application provides a culture medium which is for cryoprotecting lactic acid bacteria and contains cysteine or a salt thereof as an active ingredient.

Still another aspect of the present application provides a method for producing a lactic acid bacteria preparation, the method including: culturing lactic acid bacteria in the culture medium; collecting bacterial cells of the cultured lactic acid bacteria; and powdering the collected lactic acid bacteria cells.

Still another aspect of the present application provides a lactic acid bacteria preparation containing: lactic acid bacteria; and cysteine or a salt thereof.

According to the present application, it is possible to provide a culture medium which is for accelerating the growth of lactic acid bacteria and contains cysteine or a salt thereof as an active ingredient.

According to the present application, it is possible to provide a method for accelerating the growth of lactic acid bacteria, the method including culturing lactic acid bacteria in the culture medium according to the present application, for example, the culture medium for cryoprotecting lactic acid bacteria and/or the culture medium for accelerating the growth of lactic acid bacteria.

According to the present application, it is possible to provide a method for regulating energy metabolism of lactic acid bacteria, the method including culturing lactic acid bacteria in the culture medium according to the present application, for example, the culture medium for cryoprotecting lactic acid bacteria and/or the culture medium for accelerating the growth of lactic acid bacteria.

According to the present application, it is possible to provide a method for increasing the thermal stability of lactic acid bacteria, the method including culturing lactic acid bacteria in the culture medium according to the present application, for example, the culture medium for cryoprotecting lactic acid bacteria and/or the culture medium for accelerating the growth of lactic acid bacteria.

### ADVANTAGEOUS EFFECTS

The cysteine or a salt thereof, which is an active ingredient according to the present application, not only protects lactic acid bacteria from being frozen, but also has the effect of improving stability so that the lactic acid bacteria can maintain the activity as much as possible even after being freeze-dried to be powdered. In particular, the cysteine or a hydrochloride thereof has the effect of allowing lactic acid bacteria, which live but are no longer proliferated due to powdering, to exhibit excellent stability even in a high-temperature physical environment, and thus there is an advantage in that the distribution or preservation/storage of live lactic acid bacteria is facilitated.

Furthermore, when the lactic acid bacteria are cultured in the culture medium according to the present application, for example, the culture medium for cryoprotecting lactic acid bacteria and/or the culture medium for accelerating the growth of lactic acid bacteria, the growth yield of the lactic acid bacteria is dramatically improved, and the thermal stability of the lactic acid bacteria is dramatically improved.

However, the effects of the present application are not limited to the aforementioned effects, and other effects, which are not mentioned, will be clearly understood by a person with ordinary skill in the art from the following description.

### MODE FOR CARRYING OUT THE INVENTION

First, the terms used in the present application will be defined.

The term "lactic acid bacteria" referred to in the present application is a generic term for bacteria which acquire energy by fermenting saccharides and produce a large amount of lactic acid. The lactic acid bacteria may be one or more selected from the group consisting of *Lactobacillus sp., Bifidobacterium sp., Streptococcus sp., Lactococcus sp., Enterococcus sp., Pediococcus sp., Leuconostoc sp.,* and *Weissella sp.* The lactic acid bacteria may be specifically *Lactobacillus plantarum, Lactobacillus casei, Lactobacillus rhamnosus, Lactobacillus acidophilus, Bifidobacterium bifidum, Bifidobacterium longum, Bifidobacterium breve, Streptococcus faecalis, Lactococcus lactis subsp. lactis,* or the like, and more specifically *Lactobacillus plantarum CJLP243* disclosed in Korean Registered Patent Publication No. 1,178,217, *Lactobacillus plantarum CJLP133* disclosed in Korean Registered Patent Publication No. 1,486,999, *Lactobacillus plantarum CJLP136* disclosed in Korean Registered Patent Publication No. 1,075,558, *Lactobacillus plantarum CJLP55* disclosed in Korean Registered Patent Publication No. 1,255,050, *Lactobacillus plantarum CJLP56* disclosed in Korean Registered Patent Publication No. 1,075,557, or the like, but are not particularly limited thereto.

The term "cryoprotecting" referred to in the present application means protecting the lactic acid bacteria tissue from being frozen, when the lactic acid bacteria are freeze-dried and then preserved in order to maintain the activity thereof as it is.

The term "freeze drying" referred to in the present application is a method in which a material to be dried is frozen by rapidly lowering the temperature of a container, then the internal pressure of the container is made into a vacuum, and the solidified solvent contained in the material is immediately sublimated into water vapor to perform drying. The freeze drying is a method by which damage to a substance sensitive to heat can be minimized and the substance can be preserved for a long time, and useful in terms of contamination prevention, storage, transportation, and economic efficiency. However, the freezing temperature of the freeze drying as described above may be a sub-zero temperature such as -40°C to -196°C (boiling point of liquid nitrogen), -50°C to -196°C, or -70°C to -196°C, and when the lactic acid bacteria are freeze-dried, the activity and survival rate of the lactic acid bacteria are rapidly reduced during the process, ice particles are formed during freezing, and thus there is a problem in that the membrane structure of the lactic acid bacteria cells is damaged. A substance or composition which is added together during freeze drying so that the function can be recovered during rehydration without damaging or killing the lactic acid bacteria, in order to solve such a problem, is referred to as a "cryoprotective agent" or a "composition for cryoprotecting", and serves to impart physicochemical stability to the lactic acid bacteria, to increase the survival rate.

Hereinafter, the present application will be described in detail.

The present application provides a composition for cryoprotecting lactic acid bacteria.

The composition for cryoprotecting lactic acid bacteria according to the present application contains cysteine or a salt thereof as an active ingredient.

The cysteine is a kind of sulfur-containing α-amino acid having the structure of HS-CH₂CH(NH₂)-COOH, and has a sulfhydryl group and thus forms a disulfide bond with another cysteine. The cysteine may be L-cysteine, D-cysteine, or L,D-cysteine, and may be specifically L-cysteine.

The salt of the cysteine may be any salt of the cysteine, and may be, for example, a hydrochloride, a sulfate, or the like.

The cysteine or a salt thereof not only protects lactic acid bacteria from being frozen to minimize damage or death of the lactic acid bacteria caused by freezing, but also serves to improve the stability of the lactic acid bacteria so that the lactic acid bacteria can maintain the intrinsic activity even after being freeze-dried. Therefore, the cysteine or a salt thereof may be used not only as an active ingredient of the composition for cryoprotecting lactic acid bacteria, but also as an active ingredient of a composition for improving the stability of the lactic acid bacteria which are dried, in particular, freeze-dried.

The cysteine or a salt thereof may be contained in a content range consisting of a combination of 0.01 wt% or more, for example, one lower limit selected from the group consisting of 0.01 wt%, 0.05 wt%, and 0.1 wt%, and/or 10 wt% or less, for example, one upper limit selected from the group consisting of 10 wt%, 7 wt%, 5 wt%, and 3 wt%, with respect to 100 wt% of the total amount of the composition. For example, the cysteine or a salt thereof may be contained in an amount of 0.05 wt% to 10 wt%, specifically in an amount of 0.05 wt% to 7 wt%, 0.05 wt% to 5 wt%, or 0.05 wt% to 3 wt%, or specifically in an amount of 0.1 wt% to 10 wt%, 0.1 wt% to 7 wt%, 0.1 wt% to 5 wt%, or 0.1 wt% to 3 wt%, with respect to 100 wt% of the total amount of the composition, and the content of the cysteine or a salt thereof as described above could be appropriately adjusted by a person with ordinary skill in the art, according to the type, size, and amount of the lactic acid bacteria, freeze drying conditions, the type or content of other ingredients contained in the composition, and the like.

The composition for cryoprotecting lactic acid bacteria according to the present application may further contain a cryoprotective agent, a porous support, a nitrogen source, or the like.

The cryoprotective agent refers to a substance, excluding the cysteine or a salt thereof, which has cryoprotective efficacy commonly used in the technical field to which the present application belongs, commercially available products can be purchased and used, and the type thereof is not particularly limited. Specifically, the cryoprotective agent may be saccharides, an amino acid, a peptide, gelatin, glycerol, sugar alcohol, whey, alginic acid, ascorbic acid, a yeast extract, skim milk, or the like. For example, trehalose, which is a type of saccharides, can be used as the cryoprotective agent. The trehalose is a saccharide which is widely present in nature such as a plant and a microorganism, and is a substance known to act as a cryoprotective agent which prevents damage or death of the lactic acid bacteria caused by freeze drying and helps recover the function thereof during rehydration. The trehalose may be contained in an amount of 10 wt% to 40 wt%, for example, 10 wt% to 30 wt%, specifically 15 wt% to 25 wt%, and more specifically 17.5 wt% to 22.5 wt%, with respect to 100 wt% of the total amount of the composition for cryoprotecting lactic acid bacteria, but the content thereof could be appropriately adjusted by a person with ordinary skill in the art, according to the content of the active ingredient contained in the composition for cryoprotecting lactic acid bacteria, the type, size, and amount of the lactic acid bacteria, freeze drying conditions, the type or content of other ingredients contained in the composition, and the like.

The porous support serves to block the inflow of external moisture and air and impart porosity to freeze-dried lactic acid bacteria to facilitate a rehydration action. The porous support is a porous support commonly used during freeze drying in the technical field to which the present application belongs, commercially available products can be purchased and used, and the type thereof is not particularly limited. The porous support may be specifically maltodextrin, alginate, chitosan, starch, polyethylene glycol, propylene glycol, triacetin, acetyltriethyl citrate, triethyl citrate, glycerin, or a combination thereof, and more specifically maltodextrin. The maltodextrin is a white powder based on porous particles, is a food additive often used in general foods such as yogurt, sauce, and a salad dressing, and can also be used as a porous support during freeze-drying of lactic acid bacteria. The content of the maltodextrin may be 0.1 wt% to 20 wt%, for example, 0.5 wt% to 15 wt%, specifically 1 wt% to 10 wt%, and more specifically 2.5 wt% to 7.5 wt%, with respect to 100 wt% of the total amount of the composition for cryoprotecting lactic acid bacteria.

The nitrogen source (N-source) refers to a substance used as a nitrogen energy source for lactic acid bacteria, and serves to prevent damage to bacterial cells caused by post-fermentation. When lactic acid bacteria are mixed with the composition for cryoprotecting, lactic acid bacteria, which live in the absence of an energy source, generate an organic acid, which causes a pH to be decreased and induces the death of lactic acid bacteria. Accordingly, the nitrogen energy source prevents the generation of organic acids and the resulting decrease in a pH, and thus the death of lactic acid bacteria can be prevented. The nitrogen source is a nitrogen source commonly used during freeze drying in the technical field to which the present application belongs, commercially available products can be purchased and used, and the type thereof is not particularly limited. Specifically, the nitrogen source may be a skimmed milk powder, a whey protein, a yeast extract, a malt extract, a beef extract, a casein hydrolyzate, a malt extract, tryptone, cysteine, peptone, or the like, and for example, the peptone may be soy peptone, fish peptone, proteose peptone, casein peptone, peptone No.3, or the like, and may be representatively soy peptone. The content of the soy peptone may be 0.1 wt% to 20 wt%, for example, 0.5 wt% to 15 wt%, specifically 1 wt% to 10 wt%, and more specifically 2.5 wt% to 7.5 wt%, with respect to 100 wt% of the total amount of the composition for cryoprotecting lactic acid bacteria.

The composition for cryoprotecting lactic acid bacteria can be applied in the form of a coating agent. That is, the composition for cryoprotecting lactic acid bacteria may be mixed with lactic acid bacteria to coat the surface of the lactic acid bacteria, thereby protecting the lactic acid bacteria from the external environment and increasing preservation stability, but is not particularly limited thereto.

A lactic acid bacteria preparation produced from the composition for cryoprotecting lactic acid bacteria is as described later.

The present application provides a culture medium for cryoprotecting lactic acid bacteria.

In an aspect, the culture medium according to the present application may be a culture medium for accelerating the growth of lactic acid bacteria.

In an aspect, the culture medium according to the present application may be a culture medium for increasing the thermal stability of lactic acid bacteria.

In an aspect, the culture medium for cryoprotecting lactic acid bacteria may be a culture medium for accelerating the growth of lactic acid bacteria and increasing the thermal stability.

The culture medium refers to a mixture of nutritive substances required for culturing lactic acid bacteria, and supplies growth factors and nutritive substances, including water indispensable to the survival and growth of lactic acid bacteria.

The culture medium according to the present application contains cysteine or a salt thereof as an active ingredient.

The cysteine is a kind of sulfur-containing α-amino acid having the structure of HS-CH₂CH(NH₂)-COOH, and has a sulfhydryl group and thus forms a disulfide bond with another cysteine. The cysteine may be L-cysteine, D-cysteine, or L,D-cysteine, and may be specifically L-cysteine.

The salt of the cysteine may be any salt of the cysteine, and may be, for example, a hydrochloride, a sulfate, or the like.

The cysteine or a salt thereof not only protects lactic acid bacteria from being frozen to minimize damage or death of the lactic acid bacteria caused by freezing, but also serves to improve the stability of the lactic acid bacteria so that the lactic acid bacteria can maintain the intrinsic activity even after being freeze-dried. In addition, the cysteine or a salt thereof not only can dramatically increase the production of lactic acid bacteria during culturing of the lactic acid bacteria, but also serves to improve the stability of lactic acid bacteria so that the cultured lactic acid bacteria can maintain the intrinsic activity. Therefore, the cysteine or a salt thereof may be used not only as an active ingredient of the culture medium for cryoprotecting lactic acid bacteria, but also as an active ingredient of a culture medium for improving the stability of the lactic acid bacteria which are dried, in particular, freeze-dried, a culture medium for accelerating the growth of lactic acid bacteria, a composition for improving the stability of the lactic acid bacteria cultured in such a culture medium, or a culture medium for increasing the thermal stability of lactic acid bacteria.

The cysteine or a salt thereof may be contained in an amount of 0.05 wt% to 10 wt%, specifically in an amount of 0.05 wt% to 7 wt%, 0.05 wt% to 5 wt%, or 0.05 wt% to 3 wt%, or specifically in an amount of 0.1 wt% to 10 wt%, 0.1 wt% to 7 wt%, 0.1 wt% to 5 wt%, or 0.1 wt% to 3 wt%, with respect to 100 wt% of the total amount of the culture medium, and the content of the cysteine or a salt thereof as described above could be appropriately adjusted by a person with ordinary skill in the art, according to the type, size, and amount of the lactic acid bacteria, freeze drying conditions, the type or content of other ingredients contained in the culture medium, and the like.

The culture medium according to the present application may further contain nutritional components commonly contained in the culture medium, such as a carbon source, a nitrogen source, a phosphorus source, an inorganic compound, an amino acid, and/or a vitamin, in addition to the aforementioned active ingredients.

The carbon source may include a carbohydrate such as glucose, fructose, sucrose, maltose, mannitol, and sorbitol; an organic acid such as pyruvic acid, lactic acid, and citric acid; and an amino acid such as glutamic acid, methionine, and lysine. Moreover, a natural organic nutrient such as a starch hydrolyzate, molasses, blackstrap molasses, rice bran, cassava, sugarcane bagasse, and corn steep liquor can be used, specifically, a carbohydrate such as glucose and sterilized pretreated molasses (that is, molasses converted to reducing sugar) can be used, and an appropriate amount of other carbon sources can be used in various manners without limitation. These carbon sources may be used alone or in combination of two or more thereof, but are not limited thereto.

As the nitrogen source, an inorganic nitrogen source such as ammonia, ammonium sulfate, ammonium chloride, ammonium acetate, ammonium phosphate, ammonium carbonate, and ammonium nitrate; and an organic nitrogen source such as an amino acid such as glutamic acid, methionine, and glutamine, peptone, NZ-amine, a meat extract, a yeast extract, a malt extract, corn steep liquor, a casein hydrolyzate, fish or a decomposition product thereof, and defatted soybean cake or a decomposition product thereof can be used. These nitrogen sources may be used alone or in combination of two or more thereof, but are not limited thereto.

The phosphorus source may include monopotassium phosphate, dipotassium phosphate, a sodium-containing salt corresponding thereto, or the like. As the inorganic compound, sodium chloride, calcium chloride, iron chloride, magnesium sulfate, iron sulfate, manganese sulfate, calcium carbonate, and the like can be used, and in addition to the aforementioned substances, an amino acid, a vitamin, and/or a suitable precursor may be included. These constituent ingredients or precursors may be added to a medium batchwise or continuously, but are not limited thereto.

Furthermore, the pH of the culture medium can be adjusted by adding a compound such as ammonium hydroxide, potassium hydroxide, ammonia, phosphoric acid, and sulfuric acid to the culture medium, and the formation of bubbles during culturing can be suppressed by using an antifoaming agent such as a fatty acid polyglycol ester. Moreover, oxygen or oxygen-containing gas may be injected into the culture medium in order to maintain an aerobic state during culturing, or gas may not be injected or nitrogen, hydrogen, or carbon dioxide gas may be injected in order to maintain an anaerobic or microaerobic state, but the present application is not limited thereto.

The culture medium for accelerating the growth of lactic acid bacteria according to the present application may further contain a lactic acid bacteria growth accelerating adjuvant. The lactic acid bacteria growth accelerating adjuvant is an ingredient, excluding the cysteine or a salt thereof, which can be contained as another active ingredient that accelerates the growth of lactic acid bacteria, and is contained together with the cysteine or a salt thereof, and thus has an advantage in that an effect of accelerating the growth of lactic acid bacteria and/or an effect of improving the stability of lactic acid bacteria can be further improved. The lactic acid bacteria growth accelerating adjuvant specifically includes at least one selected from the group consisting of coenzyme Q10, vitamin C, vitamin E, and vitamin B2, and the lactic acid bacteria growth accelerating adjuvant is not limited thereto, but, for example, may be contained in an amount of 0.01 wt% to 30 wt% with respect to 100 wt% of the total amount of the culture medium, and for example, may be contained in an amount of 0.1 to 25 wt%, 0.1 to 20 wt%, 0.1 to 15 wt%, or 1 to 10 wt%. More specifically, the mixing weight ratio of the cysteine or a salt thereof to the lactic acid bacteria growth accelerating adjuvant may be 1:3 to 3:1, and, for example, may be 1:2 to 2:1, 1:1.5 to 1.5:1, or 1:1.

The culture medium may be in a solid form or a liquid form.

A lactic acid bacteria preparation produced from the culture medium is as described later.

The present application provides a method for producing a lactic acid bacteria preparation, specifically, a lactic acid bacteria preparation having thermal stability.

The method for producing a lactic acid bacteria preparation according to the present application includes mixing lactic acid bacteria with a composition containing cysteine or a salt thereof to prepare a mixture, and freeze-drying the mixture to be powdered.

In an aspect, the composition for cryoprotecting lactic acid bacteria according to the present application can be used as the composition containing cysteine or a salt thereof.

In another aspect, the culture medium according to the present application, for example, the culture medium for cryoprotecting lactic acid bacteria can be used as the composition containing cysteine or a salt thereof.

Hereinafter, the method for producing a lactic acid bacteria preparation according to the present application will be described.

First, lactic acid bacteria are mixed with a composition containing cysteine or a salt thereof, for example, the composition for cryoprotecting lactic acid bacteria.

The lactic acid bacteria and the composition for cryoprotecting lactic acid bacteria may be mixed in a ratio of 1:0.1 to 1:5, specifically 1:0.5 to 1:4, and more specifically 1:1 to 1:3, in terms of weight. Within the above mixing ratio, the lactic acid bacteria can be effectively protected from being frozen, and powdering can be efficiently performed.

The timing of mixing the lactic acid bacteria and the composition for cryoprotecting lactic acid bacteria, specifically, the timing of administering cysteine or a salt thereof in a step of culturing the lactic acid bacteria may be any timing without limitation in the step of culturing the lactic acid bacteria in the entire process of the method for producing a lactic acid bacteria preparation, and the administration is performed, for example, before culturing the lactic acid bacteria, or in at least one step of a lag phase, an exponential phase, and a stationary phase of the lactic acid bacteria.

For example, before being mixed with the composition for cryoprotecting lactic acid bacteria as described above, the lactic acid bacteria may be sufficiently cultured by conventional means and methods, and collected by a conventional method.

The culturing means that the lactic acid bacteria are grown under appropriately regulated environmental conditions. The culturing process may be performed according to suitable mediums and culture conditions known in the technical field to which the present application belongs. Such a culturing process could be appropriately adjusted by a person with ordinary skill in the art, depending on a strain to be selected. For example, the lactic acid bacteria may be cultured in the form of a batch type, a continuous type, a fed-batch type, or the like. The culture temperature of the lactic acid bacteria may be a temperature of 20°C to 50°C and specifically a temperature of 30°C to 40°C. The culture time of the lactic acid bacteria may be 1 hour to 100 hours and specifically 5 hours to 50 hours.

In the step of collecting bacterial cells of the lactic acid bacteria, the desired bacterial cells may be collected from the medium by using an appropriate method known in the technical field to which the present application belongs according to the culturing form of the lactic acid bacteria as described above. For example, centrifugal separation, filtration, a treatment with a crystallized protein precipitant (salting-out method), extraction, ultrasonic disruption, ultrafiltration, a dialysis method, various types of chromatography such as molecular sieve chromatography (gel filtration), adsorption chromatography, ion exchange chromatography, and affinity chromatography, HPLC, and a combination of these methods can be used. The collecting step may involve an additional purification process, and the collected bacterial cells of the lactic acid bacteria may be further purified by using an appropriate method known in the technical field to which the present application belongs.

Next, the mixture of the lactic acid bacteria and the composition for cryoprotecting lactic acid bacteria is freeze-dried to be powdered.

The process of powdering the mixture of the lactic acid bacteria and the composition for cryoprotecting lactic acid bacteria can be performed through freeze drying which is generally used in the food field in the related art. The freeze drying can be performed at a temperature of -70°C to 30°C and specifically a temperature of -70°C to -40°C. The freeze drying can be performed through the process of removing moisture by performing freezing under cooling conditions for 3 hours to 48 hours, specifically 6 hours to 36 hours, and more specifically 12 hours to 24 hours, and then performing thawing in a freeze dryer.

As described above, by mixing the lactic acid bacteria with the composition for cryoprotecting lactic acid bacteria according to the present application and then freeze-drying the mixture to be powdered, the stability is improved, and thus a lactic acid bacteria preparation in which the activity of the lactic acid bacteria is maintained as much as possible can be produced.

In an aspect, the method for producing a lactic acid bacteria preparation may further include mixing the lactic acid bacteria with composition for cryoprotecting lactic acid bacteria to prepare a mixture and culturing lactic acid bacteria cells using the mixture before freeze-drying the mixture.

The method for producing a lactic acid bacteria preparation according to the present application may include mixing the lactic acid bacteria with composition for cryoprotecting lactic acid bacteria, culturing bacterial cells of the lactic acid bacteria using the mixture, collecting the cultured bacterial cells of the lactic acid bacteria, and powdering the collected lactic acid bacteria cells.

The culturing means that the lactic acid bacteria are grown under appropriately regulated environmental conditions. The culturing process may be performed under suitable culture conditions known in the technical field to which the present application belongs. Such a culturing process could be appropriately adjusted by a person with ordinary skill in the art, depending on a strain to be selected. For example, the lactic acid bacteria may be performed at a temperature of 20°C to 50°C and specifically a temperature of 30°C to 40°C, in the form of a batch type, a continuous type, a fed-batch type, or the like. The culturing may be performed for 1 hour to 100 hours and specifically 5 hours to 50 hours.

Subsequently, the bacterial cells of the lactic acid bacteria cultured as described above are collected.

In the step of collecting bacterial cells of the lactic acid bacteria, the desired bacterial cells may be collected from the medium by using an appropriate method known in the technical field to which the present application belongs according to the culturing form of the lactic acid bacteria as described above. For example, centrifugal separation, filtration, a treatment with a crystallized protein precipitant (salting-out method), extraction, ultrasonic disruption, ultrafiltration, a dialysis method, various types of chromatography such as molecular sieve chromatography (gel filtration), adsorption chromatography, ion exchange chromatography, and affinity chromatography, HPLC, and a combination of these methods can be used. The collecting step may involve an additional purification process, and the collected bacterial cells of the lactic acid bacteria may be further purified by using an appropriate method known in the technical field to which the present application belongs.

The lactic acid bacteria cells collected as described above may be mixed with a composition containing a cryoprotective agent, a porous support, a nitrogen source, or the like, without cysteine or a salt thereof. The descriptions of the cryoprotective agent, the porous support, and the nitrogen source are the same as those described for the composition for cryoprotecting lactic acid bacteria.

Finally, the lactic acid bacteria cells collected as described above are powdered.

The process of powdering the collected lactic acid bacteria cells can be performed through a powdering method which is generally used in the food field in the related art, and, for example, can be performed by freeze-drying the collected lactic acid bacteria cells. The freeze drying can be performed through the process of removing moisture by performing freezing under cooling conditions at a temperature of -70°C to 30°C and specifically a temperature of -70°C to - 40°C for 3 hours to 48 hours, specifically 6 hours to 36 hours, and more specifically 12 hours to 24 hours and then performing thawing in a freeze dryer.

As described above, by culturing the lactic acid bacteria in the form of a mixture with the composition for cryoprotecting lactic acid bacteria, and powdering lactic acid bacteria cells collected therefrom, the stability is improved, and thus a lactic acid bacteria preparation in which the activity of the lactic acid bacteria is maintained as much as possible can be produced.

When the culture medium containing cysteine or a salt thereof, for example, the culture medium for cryoprotecting lactic acid bacteria is used as the composition containing cysteine or a salt thereof, the method for producing a lactic acid bacteria preparation is the same as the production method using the composition for cryoprotecting lactic acid bacteria' in the aforementioned production method.

The stability of a lactic acid bacteria preparation produced according to the method for producing a lactic acid bacteria preparation according to the present application will be described later.

Another aspect of the present application provides a method for accelerating the growth of lactic acid bacteria, a method for regulating energy metabolism of lactic acid bacteria, and a method for increasing the thermal stability of lactic acid bacteria.

The method for accelerating the growth of lactic acid bacteria, the method for regulating energy metabolism of lactic acid bacteria, and the method for increasing the thermal stability of lactic acid bacteria according to the present application may include culturing the lactic acid bacteria in the aforementioned culture medium, for example, the culture medium for accelerating the growth of lactic acid bacteria according to the present application, and/or mixing the lactic acid bacteria with the aforementioned composition for cryoprotecting lactic acid bacteria and culturing the lactic acid bacteria in the form of the mixture. The specific method thereof may be performed in the same manner as the aforementioned method for producing a lactic acid bacteria preparation.

Here, the lactic acid bacteria are cultured in the culture medium containing cysteine or a salt thereof, for example, the culture medium for accelerating the growth of lactic acid bacteria, and/or using the composition for cryoprotecting lactic acid bacteria, the energy production mechanism thereof is not limited thereto, but lactic acid bacteria can be grown by producing energy through 'breathing'.

In general, for culturing facultative anaerobic lactic acid bacteria, culture conditions are changed to conditions for minimizing oxygen exposure, such as facultative anaerobic conditions, for example, oxygen/nitrogen substitution in a fermenter, or the like is performed, but when the medium containing cysteine or a salt thereof according to the present application is used, the growth of the lactic acid bacteria can be excellently accelerated by providing an environment in which the lactic acid bacteria can remove active oxygen, through the culture medium without separately changing culture conditions.

In addition, air, for example, sterilized air is caused to appropriately flow into a culture tank to control the growth acceleration of the lactic acid bacteria.

The lactic acid bacteria of which the growth is accelerated according to the present application has improved stability, and thus has the effect of maintaining the number of lactic acid bacteria at a high level for a long period of time.

The stability of a lactic acid bacteria preparation produced according to the method for producing a lactic acid bacteria preparation according to the present application will be described later.

The present application provides a lactic acid bacteria preparation, specifically, a lactic acid bacteria preparation having thermal stability.

The lactic acid bacteria preparation according to the present application contains lactic acid bacteria, and cysteine or a salt thereof.

The cysteine is a kind of sulfur-containing α-amino acid having the structure of HS-CH₂CH(NH₂)-COOH, and has a sulfhydryl group and thus forms a disulfide bond with another cysteine. The cysteine may be L-cysteine, D-cysteine, or L,D-cysteine, and may be specifically L-cysteine.

The salt of the cysteine may be any salt of the cysteine, and may be specifically a hydrochloride, a sulfate, or the like.

The lactic acid bacteria preparation may be in the form of a granule, a powder, a powdered drug, a pellet, an infusum, an emulsion, a flow agent, a tablet, a pill, a capsule, a pellet, an ointment, a suppository, an injection, an inhalant, an aerosol, a suspension, a syrup, an emulsion, a soft capsule, a hard capsule, an elixir, a troche, or a lozenge, and may be specifically in the form of a freeze-dried powder.

Due to the cysteine or a salt thereof, the lactic acid bacteria are protected from being frozen to minimize damage or death of the lactic acid bacteria, and further, the intrinsic activity of the lactic acid bacteria can be maintained even after being produced in the form of a powder.

For example, after the lactic acid bacteria preparation according to the present application are preserved at 40°C for 4 weeks, the lactic acid bacteria preparation may show a survival rate of lactic acid bacteria of 45% or more, and specifically 50% or more, 50.5% or more, 51% or more, 55% or more, 60% or more, 65% or more, 70% or more, 75% or more, or 77% or more, compared to the initial stage of the preservation. In an aspect, after the lactic acid bacteria preparation is preserved at 40°C for 3 weeks, the lactic acid bacteria preparation may show a survival rate of lactic acid bacteria of 45% or more, and specifically 50% or more, 51% or more, 52% or more, 55% or more, 60% or more, 65% or more, 70% or more, 75% or more, 80% or more, or 81% or more, compared to the initial stage of the preservation. In an aspect, after the lactic acid bacteria preparation is preserved at 40°C for 2 weeks, the lactic acid bacteria preparation may show a survival rate of lactic acid bacteria of 55% or more, and specifically 60% or more, 62% or more, 64% or more, 65% or more, 70% or more, 75% or more, 80% or more, 85% or more, 90% or more, or 91% or more, compared to the initial stage.

The initial stage of the preservation may be day 0 of the preservation, or just before the preservation.

Moreover, due to the cysteine or a salt thereof, the growth of the lactic acid bacteria can be accelerated during culturing of the lactic acid bacteria.

For example, when the lactic acid bacteria are cultured at 37°C in the presence of cysteine or a salt thereof, and a culture solution is diluted 20 times, the optical density (O.D.) value measured using a spectrophotometer may be, after 7 hours, 0.5 or more, and specifically 0.7 or more, 0.9 or more, 1.0 or more, or 1.2 or more. In an aspect, the O.D. value after 8 hours may be 0.6 or more, 0.8 or more, 1.0 or more, 1.2 or more, or 1.3 or more. In an aspect, the O.D. value after 9 hours may be 0.7 or more, 0.8 or more, 0.9 or more, 1.0 or more, 1.2 or more, 1.3 or more, or 1.4 or more. In an aspect, the O.D. value after 10 hours may be 0.8 or more, 1.0 or more, 1.2 or more, 1.4 or more, or 1.5 or more. In an aspect, the O.D. value after 11 hours may be 1.0 or more, 1.1 or more, 1.2 or more, 1.3 or more, 1.4 or more, or 1.5 or more.

For example, when the lactic acid bacteria are cultured at 37°C in the presence of cysteine or a salt thereof, the number of live bacteria (CFU/ml) after 7 hours may be, but not limited to, 1.5×10^9 or more, 2.0×10^9 or more, 2.5×10^9 or more, 3.0×10^9 or more, 3.5×10^9 or more, 4.0×10^9 or more, 5.0×10^9 or more, or 5.5×10^9 or more. In an aspect, the number of live bacteria after 8 hours may be 2.5×10^9 or more, 3.0×10^9 or more, 3.5×10^9 or more, 4.0×10^9 or more, 5.0×10^9 or more, 5.5×10^9 or more, 6.0×10^9 or more, 6.5×10^9 or more, 7.0×10^9 or more, 7.5×10^9 or more, 8.0×10^9 or more, 8.5×10^9 or more, or 9.0×10^9 or more. In an aspect, the number of live bacteria after 9 hours may be 3.0×10^9 or more, 5.0×10^9 or more, 7.0×10^9 or more, 9.0×10^9 or more, or 9.5×10^9 or more. In an aspect, the number of live bacteria after 10 hours may be 4.0×10^9 or more, 5.0×10^9 or more, 7.0×10^9 or more, 9.0×10^9 or more, 9.5×10^9 or more, 1.0×10^10 or more, or 1.2×10^10 or more. In an aspect, the number of live bacteria after 11 hours may be 5.0×10^9 or more, 7.0×10^9 or more, 9.0×10^9 or more, 9.5×10^9 or more, 1.0×10^10 or more, or 1.2×10^10 or more.

Hereinafter, the present application will be described in detail with reference to Examples.

However, the following Examples are to specifically illustrate the present application, and the contents of the present application are not limited by the following examples.

### [Example 1]

A *Lactobacillus plantarum CJLP133* strain was cultured at 37°C for 18 to 24 hours using an MRS liquid medium (Difco, USA), then a supernatant was discarded using a centrifugal separator, and only lactic acid bacteria were collected.

The lactic acid bacteria cells collected as described above were mixed, in a weight ratio of 1:2, with a composition for cryoprotecting lactic acid bacteria, which was produced by mixing cysteine monohydrochloride, trehalose, maltodextrin, soy peptone, and water in the contents shown in Table 1 below and sterilizing the mixture.

**[Table 1]**

| **Ingredient** | **Content (wt%)** | | | |
|---|---|---|---|---|
| | **Example 1-1** | **Example 1-2** | **Example 1-3** | **Example 1-4** |
| Cysteine Monohydrochloride | 0.1 | 0.5 | 1 | 5 |
| Trehalose | 20 | 20 | 20 | 20 |
| Maltodextrin | 5 | 5 | 5 | 5 |
| Soy Peptone | 5 | 5 | 5 | 5 |
| Water | Remainder | Remainder | Remainder | Remainder |
| Total | 100 | 100 | 100 | 100 |

After the mixture was suspended, the resultant was transferred to a freeze-drying tray and maintained for 12 to 24 hours under quick freezing conditions (-40°C or lower), and then moisture was removed while performing thawing in a freeze dryer, to obtain a lactic acid bacteria powder coated with the composition of Table 1.

### [Example 2]

A *Lactobacillus plantarum CJLP133* strain disclosed in Korean Registered Patent Publication No. 1,486,999 was cultured at 37°C for 18 to 24 hours using an MRS liquid medium (Difco, USA) containing 0.1 wt% of cysteine monohydrochloride, then a supernatant was discarded using a centrifugal separator, and only lactic acid bacteria were collected.

The lactic acid bacteria cells collected as described above were mixed, in a weight ratio of 1:2, with a composition for cryoprotecting lactic acid bacteria, which was produced by mixing trehalose, maltodextrin, soy peptone, and water in the contents shown in Table 2 below and sterilizing the mixture.

**[Table 2]**

| **Ingredient** | **Content (wt%)** |
|---|---|
| Trehalose | 20 |
| Maltodextrin | 5 |
| Soy Peptone | 5 |
| Water | Remainder |
| Total | 100 |

After the mixture was suspended, the resultant was transferred to a freeze-drying tray and maintained for 12 to 24 hours under quick freezing conditions (-40°C or lower), and then moisture was removed while performing thawing in a freeze dryer, to obtain a lactic acid bacteria powder coated with the composition of Table 2.

### [Example 3]

A *Lactobacillus plantarum CJLP133* strain was cultured at 37°C for 11 hours in an MRS liquid medium (Difco, USA) containing 0.1% of cysteine monohydrochloride. The ingredients of the MRS medium containing cysteine monohydrochloride are as shown in Table 3, and the pH, which was decreased during culturing, was set to be neutralized to a pH of 5.95 through an automatic aqueous ammonia feeding system.

**[Table 3]**

| Ingredient | Content |
|---|---|
| L-Cysteine Monohydrochloride | 1 g |
| Proteose Peptone | 10 g |
| Beef Extract | 10 g |
| Yeast Extract | 5 g |
| Dextrose | 20 g |
| Polysorbate 80 | 1 g |
| Ammonium Citrate | 2 g |
| Sodium Acetate | 5 g |
| Magnesium Sulfate | 0.1 g |
| Manganese Sulfate | 0.05 g |
| Dipotassium Phosphate | 2 g |
| Water | 1,000 mL |

### [Comparative Example 1]

A *Lactobacillus plantarum CJLP133* strain was cultured at 37°C for 18 to 24 hours using an MRS liquid medium (Difco, USA), then a supernatant was discarded using a centrifugal separator, and only lactic acid bacteria were collected.

The lactic acid bacteria cells collected as described above were mixed, in a weight ratio of 1:2, with a composition for cryoprotecting lactic acid bacteria, which was produced by mixing trehalose, maltodextrin, soy peptone, and water in the contents shown in Table 2 and sterilizing the mixture.

After the mixture was suspended, the resultant was transferred to a freeze-drying tray and maintained for 12 to 24 hours under quick freezing conditions (-40°C or lower), and then moisture was removed while performing thawing in a freeze dryer, to obtain a lactic acid bacteria powder coated with the composition of Table 2.

### [Comparative Example 2]

A *Lactobacillus plantarum CJLP133* strain was cultured at 37°C for 11 hours in an MRS liquid medium (Difco, USA) not containing 0.1% of cysteine monohydrochloride. The ingredients of the MRS medium are as shown in Table 4, and the pH, which was decreased during culturing, was set to be neutralized to a pH of 5.95 through an automatic aqueous ammonia feeding system.

**[Table 4]**

| Ingredient | Content |
|---|---|
| Proteose Peptone | 10 g |
| Beef Extract | 10 g |
| Yeast Extract | 5 g |
| Dextrose | 20 g |
| Polysorbate 80 | 1 g |
| Ammonium Citrate | 2 g |
| Sodium Acetate | 5 g |
| Magnesium Sulfate | 0.1 g |
| Manganese Sulfate | 0.05 g |
| Dipotassium Phosphate | 2 g |
| Water | 1,000 mL |

### [Experimental Example 1]

Evaluation of high-temperature stability of lactic acid bacteria powder depending on presence or absence of cysteine monohydrochloride

In order to evaluate the high-temperature stability of the lactic acid bacteria powder to which cysteine monohydrochloride was applied, the survival rates in Example 1-1, Example 2, and Comparative Example 1 under the severe conditions were analyzed. The activity of the freeze-dried lactic acid bacteria powder is gradually decreased depending on the storage temperature and the storage period. The factors, which generally affect the activity, include temperature, oxygen, moisture, and the like. The freeze-dried lactic acid bacteria powder is highly hygroscopic, and thus the content thereof is significantly reduced at the initial stage of storage. In order to improve distribution storage properties, there are various methods for applying a deoxidizer to a packing material or for dehumidification, but there are many differences in the storage period ultimately depending on the degree of stability of the lactic acid bacteria powder. Therefore, the respective samples were individually packaged and preserved in an aluminum pouch in order to alleviate the hygroscopicity caused by the properties of raw materials, and after being preserved at 40°C for 4 weeks, the survival rate under the severe conditions was analyzed.

Specifically, certain amounts of the samples of the lactic acid bacteria powders produced in Example 1-1, Example 2, and Comparative Example 1 were put into aluminum pouch packages, the samples were individually packaged and sealed, and each sample was preserved for 4 weeks in an incubator at 40°C. After a predetermined period of time elapsed, the sample of the experimental group was diluted with a saline buffer at a ratio of 1:100, put into a sterilizing bag, and then homogenized. The sample subjected to serial dilution with the saline buffer was smeared on an MRS agar plate. The plate was collected, static culturing was performed for 24 hours under aerobic conditions at 37°C, then the number of bacterial cells was counted, and the results thereof are shown in Table 5 below. The numbers listed in Table 5 below indicate the survival rate (%) compared to the number of lactic acid bacteria at the initial stage.

**[Table 5]**

| Timing | Survival Rate (%) | | |
|---|---|---|---|
| | Comparative Example 1 | Example 1-1 | Example 2 |
| Initial Stage | 100.0 | 100.0 | 100.0 |
| First Week | 54.7 | 72.7 | 89.0 |
| Second Week | 35.9 | 64.9 | 91.5 |
| Third Week | 40.9 | 52.2 | 81.8 |
| Fourth Week | 38.0 | 51.0 | 77.4 |

As a result of applying severe conditions and measuring the activity of the lactic acid bacteria over time, it was found that in Example 1-1 in which the composition for cryoprotecting containing cysteine monohydrochloride was used, and Example 2 in which the culture medium containing cysteine monohydrochloride was used, a much higher survival rate of lactic acid bacteria was exhibited, compared to Comparative Example 1 in which cysteine monohydrochloride was not contained.

### [Experimental Example 2]

### Evaluation of high-temperature stability of lactic acid bacteria powder according to content of cysteine monohydrochloride

In order to evaluate the high-temperature stability of the lactic acid bacteria powder, to which cysteine monohydrochloride was applied, according to the content of cysteine monohydrochloride, the survival rates of the lactic acid bacteria powders produced in Example 1-1 to Example 1-4 under the severe conditions were analyzed. The severe conditions were applied in the same manner as in Experimental Example 1, and the activity of the lactic acid bacteria over time was measured, and the results thereof are shown in Table 6 below. The numbers listed in Table 6 below also indicate the survival rate (%) compared to the number of lactic acid bacteria at the initial stage.

**[Table 6]**

| **Timing** | **Survival Rate (%)** | | | |
|---|---|---|---|---|
| | **Example 1-1** | **Example 1-2** | **Example 1-3** | **Example 1-4** |
| Initial Stage | 100.0 | 100.0 | 100.0 | 100.0 |
| First Week | 72.7 | 93.0 | 89.2 | 91.7 |
| Second Week | 64.9 | 75.2 | 82.3 | 88.5 |
| Third Week | 52.2 | 74.3 | 78.7 | 81.1 |
| Fourth Week | 51.0 | 73.4 | 77.5 | 75.5 |

### [Experimental Example 3]

### Evaluation of high-concentration culturing of lactic acid bacteria depending on presence or absence of cysteine monohydrochloride in culture medium (O.D. value)

The evaluation of the lactic acid bacteria culturing was measured by diluting a lactic acid bacteria culture solution 20 times at 600 nm using a spectrophotometer (NanoPhotometer, IMPLEN) according to the method for measuring an optical density (O.D.) value of Korean Food Additives Codex.

**[Table 7]**

| **O.D.** | **Comparative Example 2** | **Example 3** |
|---|---|---|
| 0 hr | 0 | 0 |
| 7 hr | 0.446 | 1.282 |
| 8 hr | 0.536 | 1.396 |
| 9 hr | 0.664 | 1.457 |
| 10 hr | 0.792 | 1.528 |
| 11 hr | 0.937 | 1.510 |

As can be seen in Table 7, it was found that in Example 3 in which culturing is performed in the culture medium containing cysteine monohydrochloride, the culturing rate of lactic acid bacteria was remarkably high, compared to Comparative Example 2 in which culturing is performed in the culture medium not containing cysteine monohydrochloride. From the results, it was confirmed that the ability to accelerate the growth of lactic acid bacteria due to cysteine monohydrochloride was excellent.

### [Experimental Example 4]

### Evaluation of high-concentration culturing of lactic acid bacteria depending on presence or absence of cysteine monohydrochloride in culture medium (measurement of number of live bacteria)

A lactic acid bacteria culture solution was diluted with sterilized physiological water and smeared so that 30 to 300 colonies were formed in an MRS agar plate medium, and then cultured at 37°C for 24 hours. The number of colonies observed after the culturing was counted and calculated as the number of live bacteria per mL.

**[Table 8]**

| CFU/ml | Comparative Example 2 | Example 3 |
|---|---|---|
| 0 hr | 0 | 0 |
| 7 hr | 1.2×10^9 | 5.9×10^9 |
| 8 hr | 2.0×10^9 | 9.2×10^9 |
| 9 hr | 2.8×10^9 | 1.0×10^10 |
| 10 hr | 3.3×10^9 | 1.2×10^10 |
| 11 hr | 4.2.×10^9 | 1.2×10^10 |

It was found that in Example 3 in which culturing is performed in the culture medium containing cysteine monohydrochloride, the number of live lactic acid bacteria was remarkably high, compared to Comparative Example 2 in which culturing is performed in the culture medium not containing cysteine monohydrochloride. From the results, it was confirmed that the ability to accelerate the growth of lactic acid bacteria due to cysteine monohydrochloride was excellent. Hereinbefore, the preferred Examples of the present application have been exemplarily described, but the scope of the present application is not limited to only the specific Examples described above, and could be appropriately modified by a person with ordinary skill in the art within the scope described in the claims of the present application.

## Claims

1. A composition for cryoprotecting lactic acid bacteria, comprising cysteine or a salt thereof as an active ingredient.

2. The composition for cryoprotecting lactic acid bacteria of claim 1, wherein the salt of the cysteine is a hydrochloride.

3. The composition for cryoprotecting lactic acid bacteria of claim 1, wherein the cysteine or a salt thereof is contained in an amount of 0.01 wt% to 10 wt%

4. The composition for cryoprotecting lactic acid bacteria of claim 1, further comprising at least one selected from the group consisting of trehalose, maltodextrin, and soy peptone.

5. The composition for cryoprotecting lactic acid bacteria of any one of claims 1 to 4, which has the thermal stability of lactic acid bacteria.

6. A culture medium for cryoprotecting lactic acid bacteria, comprising cysteine or a salt thereof as an active ingredient.

7. The culture medium for cryoprotecting lactic acid bacteria of claim 6, wherein the cysteine or a salt thereof is contained in an amount of 0.01 wt% to 10 wt%.

8. The culture medium for cryoprotecting lactic acid bacteria of claim 6, further comprising at least one selected from the group consisting of coenzyme Q10, vitamin C, vitamin E, and vitamin B2.

9. The culture medium for cryoprotecting lactic acid bacteria of claim 6, which further has at least one efficacy of the growth acceleration or increase in thermal stability of lactic acid bacteria.

10. A method for producing a lactic acid bacteria preparation, comprising:
mixing lactic acid bacteria with a composition containing cysteine or a salt thereof to prepare a mixture; and
freeze-drying the mixture to be powdered.

11. The method for producing a lactic acid bacteria preparation of claim 10, further comprising culturing bacterial cells of the lactic acid bacteria using the mixture before freeze-drying the mixture.

12. A lactic acid bacteria preparation having thermal stability, comprising:
lactic acid bacteria; and
cysteine or a salt thereof.

13. The lactic acid bacteria preparation of claim 12, wherein the lactic acid bacteria preparation is in the form of a granule, a powder, a powdered drug, a pellet, an infusum, an emulsion, a flow agent, a tablet, a pill, a capsule, an ointment, a suppository, an injection, an inhalant, an aerosol, a suspension, a syrup, an emulsion, a soft capsule, a hard capsule, an elixir, a troche, or a lozenge.

14. The lactic acid bacteria preparation of claim 12, wherein the lactic acid bacteria preparation is in the form of a freeze-dried powder.

15. The lactic acid bacteria preparation of any one of claims 12 to 14, wherein the survival rate of the lactic acid bacteria after the lactic acid bacteria preparation is preserved at 40°C for 4 weeks is 45% or more.

16. A method for accelerating the growth of lactic acid bacteria, comprising culturing lactic acid bacteria in the culture medium according to any one of claims 6 to 9.

17. A method for increasing the thermal stability of lactic acid bacteria, comprising culturing lactic acid bacteria in the culture medium according to any one of claims 6 to 9.
